# EUROPEAN PATENT SPECIFICATION

(11) **EP 0 724 571 B1**
(45) Date of publication and mention of the grant of the patent: **30.01.2002**
(21) Application number: 94931810.9
(22) Date of filing: 11.10.1994
(51) Int. Cl.: C07D 213/89, A61K 31/44, A61K 7/06

(54) **SUBSTITUTED PYRIDINE-2-ONES AND SUBSTITUTED PYRIDINE-2-THIONES AS BIOCIDES**
SUBSTITUIERTE PYRIDIN-2-ONE UND SUBSTITUIERTE PYRIDIN-2-THIONE ALS BIOCIDE
PYRIDINE-2-ONES SUBSTITUEES ET PYRIDINE-2-THIONES SUBSTITUEES UTILISEES EN TANT QUE BIOCIDES

(30) Priority: 18.10.1993 US 137521; 30.09.1994 US 315636
(43) Date of publication of application: 07.08.1996
(73) Proprietor: Arch Chemicals, Inc., Norwalk, CT 06856-5204 (US)
(72) Inventor: HANI, Rahim, Cheshire, CT 06410 (US); BERKOWITZ, Phillip, T., Woodbridge, CT 06525 (US)
(74) Representative: Nachshen, Neil Jacob
(86) International application number: US9411484
(87) International publication number: WO9511233

(56) References cited:
- DE-A- 2 043 797
- DE-A- 2 104 017
- US-A- 2 686 786
- US-A- 3 862 305
- US-A- 4 401 770
- US-A- 4 818 436
- US-A- 4 916 228
- CHIBA DAIGAKU KOGAKUBU KENKYU HOKOKU, vol. 31, no. 60, 1980, pages 313-319, XP002005695 TANAKA S. ET. AL.: "Structure Determination of Photoproducts by Mass Spectra Fragmentation of N-Hydroxy Lactams"
- JOURNAL OF MEDICINAL CHEMISTRY, Vol. 17, No. 7, issued 1974, DITMAR et al., "Quantitative Structure Activity Analysis in a Series of Antimycotically Active N-Hydroxy Pyridones", pages 753-756.

## Description

This invention relates to three novel 1-hydroxy-6-substituted pyridine-2-ones and 2-thiones, processes for their preparation, and their use as biocides. These compounds exhibit good biocidal activity, particularly antifungal activity.

Compounds exhibiting biocidal activity are well known in the art. For example, pyrithione salts, such as zinc pyrithione, are known to provide excellent biocidal activity, including broad spectrum anti-bacterial and anti-fungal activity. There are many uses for these pyrithiones. By way of illustration, U.S. Patent No. 4,818,436 discloses the use of pyrithiones in metalworking fluids, U.S. Patent No. 4,401,770 discloses urethane shoe inserts having antimicrobial activity; and U.S. Patent No. 4,935,061 discloses their use in paints.

Despite the excellent biocidal (particularly fungicidal) activity attributable to pyrithione salts, these compounds do have drawbacks for certain applications, most notably limited solubility in certain organic solvents and aqueous media. Accordingly, new compounds exhibiting excellent biocidal activity, but also exhibiting good solubility in organic solvents would be useful to the biocides manufacturing community.

One compound that exhibits good biocidal efficacy and solubility in shampoo formulations is 1-hydroxy-4-methyl-6-(2,4,4-trimethylpentyl)-2-pyridone. Unfortunately, this compound is more expensive to manufacture than otherwise might be desired. This compound and related compounds are disclosed in U.S. Patent 3,883,545; their use in combating dandruff is described in U.S. Patent 4,185,106; and their use in an antidandruff shampoo is described in U.S. Patent 4,711,775. U.S. Patent 4,916,228 discloses the preparation of a broad class of 1-hydroxy-2-pyridones by a process comprising reacting a pyrone having a specified structural formula with a hydroxylammonium salt in the presence of at least one alkali metal carbonate and/or hydrogen carbonate.

A technical journal publication entitled "Quantitative Structure-Activity Analysis in a Series of Antimycotically Active N-Hydroxypyridones", Journal of Medicinal Chemistry, 1974, Vol. 17, No. 7, p. 753, describes a variety of 1-hydroxy-substituted-2-pyridones, including those having para-substitutions of -SCH₂C₆H₄Cl (compound 15 in Table II thereof) and -OCH₂C₆H₄Cl (compound 16 in Table II thereof), as having antimycotic activity. However, the method of preparation of these compounds is not described in this technical publication, and it is believed that these compounds were made from 6-chloro-2-pyrone which is not commercially available.

Other new biocides providing desired solubility characteristics, as well as new processes for their preparation, would be highly desired by the biocides manufacturing community.

In one aspect, the present invention relates to a compound selected from 1-hydroxy-6-(3,5,5-trimethylhexyloxy)-pyridine-2(1H)-one, 1-hydroxy-6-octyloxypyridine-2(1H)-one and 1-hydroxy-6-octyloxypyridine-2(1H)-thione.

In a preferred embodiment, the compound is selected from 1-hydroxy-6-(3,5,5-trimethylhexyloxy)-pyridine-2(1H)-one and 1-hydroxy-6-octyloxypyridine-2(1H)-one.

In another aspect, the present invention relates to a process for making 1-hydroxy-6-(3,5,5-trimethylhexyloxy)-pyridine-2(1H)-one, characterised by the steps of:
(a) reacting 2,6-dichloro-pyridine N-oxide and 3,5,5-trimethyl-1-hexanol and a base, optionally in the presence of water or an organic solvent, at an elevated temperature to produce 2-chloro-6-(3,5,5-trimethylhexyloxy)-pyridine N-oxide, and
(b) reacting said 2-chloro-6-(3,5,5-trimethylhexyloxy)-pyridine N-oxide with a base to produce 1-hydroxy-6-(3,5,5-trimethylhexyloxy)-pyridine-2(1H)-one.

In a further aspect, the invention relates to a process for making 1-hydroxy-6-octyloxypyridine-2(1H)-one, characterised by the steps of:
(a) reacting 2,6-dichloro-pyridine N-oxide and 1-octanol and a base, optionally in the presence of water or an organic solvent, at an elevated temperature to produce 2-chloro-6-octyloxypyridine N-oxide, and
(b) reacting said 2-chloro-6-octyloxypyridine N-oxide with a base to produce 1-hydroxy-6-octyloxypyridine-2(1H)-one.

In an additional aspect, the invention relates to a method of preparing the compound 1-hydroxy-6-octyloxypyridine-2(1H)-thione, characterised by the steps of:
(a) reacting 2,6-dichloro-pyridine N-oxide and 1-octanol and a base, optionally in the presence of water or an organic solvent, at an elevated temperature to produce 2-chloro-6-octyloxypyridine N-oxide, and
(b) reacting said 2-chloro-6-octyloxypyridine N-oxide with a sulfide or hydrosulfide salt to produce compound 1-hydroxy-6-octyloxypyridine-2(1H)-thione.

The above processes can be carried out sequentially or simultaneously in a single step. Surfactants and/or phase transfer catalysts are optionally employed to facilitate the step (a) reaction in any of these processes.

In yet another aspect, the present invention relates to an antimicrobial composition comprising a functional component selected from the group consisting of paints, adhesives, coatings, elastomers, sealants, shampoos, skin care medicaments and metalworking fluid plus an antimicrobially effective amount of a compound selected from 1-hydroxy-6-(3,5,5-trimethylhexyloxy)-pyridine-2(1H)-one, 1-hydroxy-6-octyloxypyridine-2(1H)-one and 1-hydroxy-6-octyloxypyridine-2(1H)-thione or salts thereof. Illustrative salts include the amine salts, alkali metal and alkaline earth metal salts and the like.

In still another aspect, the invention relates to a method for inhibiting the growth of microorganisms by contacting said microorganisms with a composition containing an antimicrobial effective amount of a compound selected from 1-hydroxy-6-(3,5,5-trimethylhexyloxy)-pyridine-2(1H)-one, 1-hydroxy-6-octyloxypyridine-2(1H)-one and 1-hydroxy-6-octyloxypyridine-2(1H)-thione, a salt thereof, or a combination thereof, and at least one component selected from the group consisting of soaps, shampoos, skin care medicaments, cosmetics, and other polymer compounds such as adhesives, coatings, elastomers, sealants and paints.

These and other aspects will become apparent upon reading the following detailed description of the invention.

It has now been surprisingly discovered that a straightforward process can be employed to produce the 1-hydroxy-6-substituted-2-pyridones or 1-hydroxy-6-substituted-2-thione of the present invention, utilizing readily available starting materials. These compounds provide excellent antimicrobial efficacy and are inexpensive to produce.

The reactions of the processes of this invention are suitably conducted at atmospheric pressure, although higher or lower pressures may be used if desired. Suitable reaction temperatures for steps (a) and (b) range between a temperature of about 25°C to about 150°C, preferably employing a reflux temperature of between about 70°C and about 90°C. Total reaction time for the process of this invention can vary over a wide range, but is advantageously between 30 minutes and 5 hours for step (a) and between thirty minutes and 5 hours for step (b).

The reactions of the processes of this invention are suitably carried out in the presence of a base and an organic solvent. Suitable bases include alkali metal hydroxides such as sodium hydroxide or potassium hydroxide, alkaline earth metal hydroxides such as calcium or magnesium hydroxide, pyridine, triethylamine and other tertiary amine bases, potassium carbonate, "DABCO" amine catalyst (1,4-diazabicyclo(2.2.2)octane), "DBU" (1,8-diazabicyclo(5.4.0)undec-7-ene), "DBN" (1,5-diazabicyclo(4.3.0)non-5-ene, t-butyltetramethyl-guanidine, combinations thereof, and the like. Preferred bases for step (a) are anhydrous, granular or powdered sodium or potassium hydroxide, and preferred bases for step (b) are concentrated aqueous solutions of sodium or potassium hydroxide having a concentration of base of between about 10% and about 90% by weight based upon the weight of the solution. Preferably, the base is employed in at least an amount equal to the number of moles for each reactant, and a molar excess of base relative to each reactant can be employed as desired.

The reactions of the processes of this invention are suitably carried out in the presence of a solvent. Suitable organic solvents include, for example, ether, and acetone, methylene chloride, benzene, toluene, pyridine, tetrahydrofuran ("THF"), acetonitrile, dimethylsulfoxide ("DMSO"), dimethylformamide ("DMF"), and combinations thereof. Although the preferred solvent is acetonitrile, it is more preferred to run the reactions neat. As another alternative, a reactive solvent can be employed, if desired, for example by using an organic alcohol, e.g. isopropanol, or n-octanol, in an amount sufficient to provide the desired reactant and solvent characteristics.

The molar ratio of reactants for the processes of this invention can vary over a wide range, but is preferably between 10:1 and 1:10, more preferably between 2:1 and 1:2, most preferably about 1:1.

The antimicrobial compositions of this invention suitably comprise an antimicrobially effective amount of a compound as defined above, and combinations thereof, and at least one component selected from the group consisting of soaps, shampoos, skin care medicaments, cosmetics and paints. The antimicrobial compound can be employed as is or in the form of a salt, such as an amine salt such as the monoethanolamine ("MEA") salt, and the use of the salt form is preferred. By the term "antimicrobial effective amount" is meant an amount sufficient to impart to the compositions resistance against microbial attack by fungi and/or bacteria. Preferably the antimicrobial compounds are employed in the composition in a total amount of between about .01 and about 10 weight percent, more preferably between about .01 and about 5 weight percent, based upon the total weight of the composition. A particularly preferred use for the biocidal compounds of the present invention is in personal care products such as in shampoos and other hair care products, as well as in paint, paint bases and polymer compositions such as adhesive coatings, sealants, elastomers, acids and the like.

With respect to paints improved organic solubility and biocidal efficacy associated with the compounds of the present invention are expected to provide advantages when used in a wide variety of paints, including indoor and outdoor household paints, industrial and commercial paints and in particular marine paints for use, for example, or ships hulls.

Typically, a paint composition will contain a resin, a pigment and various optional additives such as thickening agent(s), wetting agents and the like, as is well known in the art. The resin is preferably selected from the group consisting of vinyl, alkyl, epoxy, acrylic, polyurethane and polyester resins, and combinations thereof. The resin is preferably employed in an amount of between about 20% and about 80% based upon the weight of the paint or paint base.

In addition, the paint composition of the present invention contains optional additional additives which have a favorable influence on the viscosity, the wetting power and the dispersibility, as well as on the stability to freezing and electrolytes and on the foaming properties. If a marine paint is being fabricated, the paint preferably contains a co-biocide such as cuprous oxide or copper thio-cyanate, a swelling agent to cause the paint to gradually "slough off" in its marine environment, thereby causing renewed biocidal efficacy of newly exposed biocide at the surface of the paint in contact with the water medium of the marine environment. Illustrative swelling agents are naturally-occurring or synthetic clays, such as kaolin, montomorillonite (bentonite, clay mica (muscovite), and chlorite (hectonite), and the like. In addition to clays, other swelling agents, including natural or synthetic polymers, such as that commercially available as POLYMERGEL, have been found to be useful in the compositions of the present invention to provide the desired "sloughing off" effect. Swelling agents can be used singly or in combination. The total amount of optional additives is preferably no greater than 20% by weight, more preferably between about 1% and about 5% by weight, based upon the total weight of the paint composition.

Illustrative thickening agents include cellulose derivatives, for example methyl, hydroxyethyl, hydroxypropyl and carboxymethyl cellulose, poly(vinyl alcohol), poly (vinylpyrolidone), poly(ethylene-glycol), salts of poly(acrylic acid) and salts of acrylic acid/acrylamide copolymers.

Suitable wetting and dispersing agents include sodium polyphosphate, salts of low-molecular-weight poly(acrylic acid), salts of poly(ethane-sulfonic acid), salts of poly (vinyl-phosphonic acid), salts of poly(maleic acid) and salts of copolymers of maleic acid with ethylene, 1-olefins with 3 to 18 carbon atoms and/or styrene.

In order to increase the stability to freezing and electrolytes there may be added to the paint composition various monomer 1,2-diols, for example glycol, propylene-glycol-(1,2), and butylene-glycol-(1,2) or polymers thereof, or ethoxylated compounds, for example reaction products of ethylene oxide with long-chain alkanols, amines, carboxylic acids, carboxylic acid amides, alkyd phenols, poly(propylene-glycol) or poly(butylene-glycol). The minimum temperature of film formation (white point) of the paint composition may be reduced by adding solvents, such as ethylene-glycol, butyl-glycol, ethyl-glycol acetate, ethyl-diglycol acetate, butyl-diglycol acetate, benzene or alkylated aromatic hydrocarbons. As defoaming agents there are suitable for example poly(propylene-glycol) and polysiloxanes.

The compounds of the present invention have many desirable attributes. They possess good antimicrobial activity and are compatible with components of conventional soaps, shampoos, skin-care medicaments, plastics and other polymer compositions and the like. These polymers are also non-volatile, hydrolytically-stable, thermally-stable and may be soluble in water and organic solvents. Furthermore, they form no undesirable colors in typical personal care items. Still further, they are expected to be favorably cost competitive with known antimicrobial additives used in conventional skin care formulations.

The following examples are intended to illustrate the present invention. In the examples, the term "g" denotes grams, "mol" denotes moles, and all percents are given on a weight basis unless otherwise specified.

### EXAMPLES

### Example 1

### Preparation of 1-Hydroxy-6-(3,5,5-trimethylhexyloxy)-pyridine-2(1H)-one

The 0.82 g (0.0050 moles) of 2,6-dichloro-pyridine N-oxide and 0.80 g (90%) (0.0050 moles) of 3,5,5-trimethyl-1-hexanol was reacted with 0.206 g (0.0050 moles) of ground sodium hydroxide in 8.2 ml of DMSO at 80°C for 8.5 hours to give 2-chloro-6-(3,5,5-trimethylhexyloxy)pyridine N-oxide. It was reacted with 0.600 g (0.015 moles) of ground sodium hydroxide at 80°C for 4 hours to give 1-hydroxy-6-(3,5,5-trimethy-hexyloxy) pyridine-2(1H)-one. After cooling, it was added 74 ml of water and was adjusted with 6N HCL to pH 3. The precipitate was filtered and washed with water to give 0.577 g (45%). It was recrystallized from ethyl acetate and hexanes: mp 130.5-131°C.

### Example 2

### Preparation of 1-Hydroxy-6-octyloxypyridine-2(1H)-one

The 0.82 g (0.0050 moles) of 2,6-dichloro-pyridine N-oxide and 0.658 g (99%) (0.0050 moles) of 1-octanol was reacted with 0.200 g (0.0050 moles) of ground sodium hydroxide in 8.2 ml of DMSO at 80°C for 4.5 hours to give 2-chloro-6-octyloxypyridine N-oxide. It was reacted with 0.600 g (0.015 moles) of ground sodium hydroxide at 80°C for 2.5 hours to give 1-hydroxy-6-octyoxypyridine-2(1H)-one. After cooling, it was added 74 ml of water and was adjusted with 6N HCL to pH 3. The precipitate was filtered and washed with water to give 0.577 g (48%). It was recrystallized from ethanol and hexanes: mp 119-120°C.

### Example 3

### Preparation of 1-hydroxy-6-octyloxypyridine-2(1H)-thione

2-Chloro-6-octyloxypyridine-N-oxide was prepared according to Example 2 and subsequent reaction with NaSH in acetonitrile and extraction with methylene chloride yielded the product as white powder.

### Determination of the Minimum Inhibitory Concentrations (MIC's) for Antimicrobial Compounds of this Invention

Solutions of the experimental compounds in dimethyl sulfoxide were serially diluted in nutrient broth (Tryptic Soy Broth for bacteria and Sabouraud Dextrose Broth for fungi) in microliter plates. Equal volumes of a broth suspension of bacteria (10⁶ CFU/ml) or fungi (10⁵ cells or spores/ml) were added to each dilution, and the plates were incubated at 37°C (bacteria and yeast) or 28°C (molds).
Bacteria, yeast and molds were incubated two, five and seven days respectively before determining the highest inhibitory dilution.

**TABLE 1**

| **MIC(ppm)** | | | | | | |
|---|---|---|---|---|---|---|
| **MIC of the 1-hydroxy-6-substituted-2-pyridones** | | | | | | |
| Compound | A | B | C | D | E | F |
| Example 1 | 32 | 32 | 2 | 2 | 16 | 2 |
| Example 2 | 32 | 16 | 4 | 2 | 32 | 4 |
| Example 3 | 16 | 16 | 4 | 4 | 8 | 16 |
| OCTOPIROX® biocide | 128 | 64 | 2 | 2 | 2 | 16-32 |
| A=Escherichia coli B=Staphylococcus aureus C=Candida albicans D=Fusarium sp. E=Aspergillus niger F=Aureobasidium pullulans "nt" denotes not tested | | | | | | |

The results provided in Table 1 show that the compounds of the present invention provide MIC's that are sometimes better than those provided by a commercial biocide, OCTOPIROX® biocide, a product of Hoechst Company. Note that the compounds of Examples 1, 2 and 3 provide particularly excellent MIC results.

## Claims

1. A compound selected from 1-hydroxy-6-(3,5,5-trimethylhexyloxy)-pyridine-2(1H)-one, 1-hydroxy-6-octyloxypyridine-2(1H)-one and 1-hydroxy-6-octyloxypyridine-2(1H)-thione.

2. A compound according to claim 1 selected from 1-hydroxy-6-(3,5,5-trimethylhexyloxy)-pyridine-2(1H)-one and 1-hydroxy-6-octyloxypyridine-2(1H)-one.

3. A process for making 1-hydroxy-6-(3,5,5-trimethylhexyloxy)-pyridine-2(1H)-one, **characterised by** the steps of:
(a) reacting 2,6-dichloro-pyridine N-oxide and 3,5,5-trimethyl-1-hexanol and a base, optionally in the presence of water or an organic solvent, at an elevated temperature to produce 2-chloro-6-(3,5,5-trimethylhexyloxy)-pyridine N-oxide, and
(b) reacting said 2-chloro-6-(3,5,5-trimethylhexyloxy)-pyridine N-oxide with a base to produce 1-hydroxy-6-(3,5,5-trimethylhexyloxy)-pyridine-2(1H)-one.

4. A process for making 1-hydroxy-6-octyloxypyridine-2(1H)-one, **characterised by** the steps of:
(a) reacting 2,6-dichloro-pyridine N-oxide and 1-octanol and a base, optionally in the presence of water or an organic solvent, at an elevated temperature to produce 2-chloro-6-octyloxypyridine N-oxide, and
(b) reacting said 2-chloro-6-octyloxypyridine N-oxide with a base to produce 1-hydroxy-6-octyloxypyridine-2(1H)-one.

5. A method of preparing the compound 1-hydroxy-6-octyloxypyridine-2(1H)-thione, **characterised by** the steps of:
(a) reacting 2,6-dichloro-pyridine N-oxide and 1-octanol and a base, optionally in the presence of water or an organic solvent, at an elevated temperature to produce 2-chloro-6-octyloxypyridine N-oxide, and
(b) reacting said 2-chloro-6-octyloxypyridine N-oxide with a sulfide or hydrosulfide salt to produce compound 1-hydroxy-6-octyloxypyridine-2(1H)-thione.

6. An antimicrobial composition comprising a functional component selected from the group consisting of paints, shampoos, skin care medicaments, metalworking fluids, polymer compositions plus an antimicrobially effective amount of a compound selected from 1-hydroxy-6-(3,5,5-trimethylhexyloxy)-pyridine-2(1H)-one, 1-hydroxy-6-octyloxypyridine-2(1H)-one and 1-hydroxy-6-octyloxypyridine-2(1H)-thione.

7. A compound selected from 1-hydroxy-6-(3,5,5-trimethylhexyloxy)-pyridine-2(1H)-one, 1-hydroxy-6-octyloxypyridine-2(1H)-one and 1-hydroxy-6-octyloxypyridine-2(1H)-thione for use in inhibiting growth of microorganisms by contacting said microorganisms with a composition **characterised by** containing an antimicrobial effective amount of said compound and at least one component selected from the group consisting of soaps, adhesives, coatings, elastomers, sealants, shampoos, skin care medicaments, cosmetics and paints.

8. A composition **characterised by** containing an antimicrobial effective amount of a compound selected from 1-hydroxy-6-(3,5,5-trimethylhexyloxy)-pyridine-2(1H)-one, 1-hydroxy-6-octyloxypyridine-2(1H)-one and 1-hydroxy-6-octyloxypyridine-2(1H)-thione and at least one component selected from the group consisting of soaps, adhesives, coatings, elastomers, sealants, shampoos, skin care medicaments, cosmetics and paints, for inhibiting the growth of microorganisms.

## Patentansprüche

1. Verbindung, die ausgewählt ist unter 1-Hydroxy-6-(3,5,5-trimethylhexyloxy)-pyridin-2(1H)-on, 1-Hydroxy-6-octyloxypyridin-2(1H)-on und 1-Hydroxy-6-octyloxypyridin-2(1H)-thion.

2. Verbindung nach Anspruch 1, die ausgewählt ist unter 1-Hydroxy-6-(3,5,5-trimethylhexyloxy)-pyridin-2(1H)-on und 1-Hydroxy-6-octyloxypyridin-2(1H)-on.

3. Verfahren zur Herstellung von 1-Hydroxy-6-(3,5,5-trimethylhexyloxy)-pyridin-2(1H)-on, **gekennzeichnet durch** folgende Schritte:
(a) Zur Reaktion bringen von 2,6-Dichlor-pyridin-N-oxid und 3,5,5-Trimethyl-1-hexanol und einer Base, gewünschtenfalls in Anwesenheit von Wasser oder eines organischen Lösungsmittels, bei erhöhter Temperatur, um 2-Chlor-6-(3,5,5-trimethylhexyloxy)-pyridin-N-oxid zu erzeugen, und
(b) zur Reaktion bringen des 2-Chlor-6-(3,5,5-trimethylhexyloxy)-pyridin-N-oxids mit einer Base, um 1-Hydroxy-6-(3,5,5-trimethylhexyloxy)-pyridin2(1H)-on zu erzeugen.

4. Verfahren zur Herstellung von 1-Hydroxy-6-octyl-oxypyridin-2(1H)-on, folgende Schritte aufweisend:
(a) Zur Reaktion bringen von 2,6-Dichlor-pyridin-N-oxid und 1-Octanol und einer Base, gewünschtenfalls in Anwesenheit von Wasser oder eines organischen Lösungsmittels, bei erhöhter Temperatur, um 2-Chlor-6-octyloxypyridin-N-oxid zu erzeugen, und
(b) zur Reaktion bringen des 2-Chlor-6-octyloxypyridin-N-oxids mit einer Base, um 1-Hydroxy-6-octyloxypyridin-2(1H)-on zu erzeugen.

5. Verfahren zur Herstellung der Verbindung 1-Hydroxy-6-octyloxypyridin-2(1H)-thion, **gekennzeichnet durch** folgende Schritte:
(a) Zur Reaktion bringen von 2,6-Dichlor-pyridin-N-oxid und 1-Octanol und einer Base, gewünschtenfalls in Anwesenheit von Wasser oder eines organischen Lösungsmittels, bei erhöhter Temperatur, um 2-Chlor-6-octyloxypyridin-N-oxid zu erzeugen, und
(b) zur Reaktion bringen des 2-Chlor-6-octyloxypyridin-N-oxids mit einem Sulfid- oder Hydrogensulfid-Salz, um die Verbindung 1-Hydroxy-6-octyloxypyridin-2(1H)-thion zu erzeugen.

6. Antimikrobielle Zusammensetzung, aufweisend einen funktionsbezogenen Bestandteil, der ausgewählt ist aus der Gruppe, die besteht aus Anstrichmitteln, Shampoos, Hautpflege-Arzneimitteln, Metallbearbeitungs-Fluiden, Polymer-Zusammensetzungen, plus eine antimikrobiell wirksame Menge einer Verbindung, die ausgewählt ist aus 1-Hydroxy-6-(3,5,5-trimethyl-hexyloxy)-pyridin-2(1H)-on, 1-Hydroxy-6-octyloxypyridin-2(1H)-on und 1-Hydroxy-6-octyloxypyridin-2(1H)-thion.

7. Verbindung, die ausgewählt ist aus 1-Hydroxy-6-(3,5,5-trimethylhexyloxy)-pyridin-2(1H)-on, 1-Hydroxy-6-octyloxypyridin-2(1H)-on und 1-Hydroxy-6-octyloxypyridin-2(1H)-thion, zur Verwendung bei der Wachstumshemmung von Mikroorganismen, indem die Mikroorganismen mit einer Zusammensetzung, die **dadurch gekennzeichnet ist, daß** sie eine antimikrobiell wirksame Menge der Verbindung und mindestens einen Bestandteil, der ausgewählt ist aus der aus Seifen, Klebstoffen, Beschichtungen, Elastomeren, Dichtmitteln, Shampoos, Hautpflege-Arzneimitteln, Kosmetika und Anstrichmitteln bestehenden Gruppe, enthält, in Berührung gebracht werden.

8. Zusammensetzung, **dadurch gekennzeichnet, daß** sie eine antimikrobiell wirksame Menge einer Verbindung, die ausgewählt ist aus 1-Hydroxy-6-(3,5,5-trimethylhexyloxy)-pyridin-2(1H)-on, 1-Hydroxy-6-octyloxypyridin-2(1H)-on und 1-Hydroxy-6-octyloxypyridin-2(1H)-thion, und mindestens einen Bestandteil, der ausgewählt ist aus der Gruppe, die besteht aus Seifen, Klebstoffen, Beschichtungen, Elastomeren, Dichtmitteln, Shampoos, Hautpflege-Arzneimitteln, Kosmetika und Anstrichmitteln, enthält, zur Hemmung des Wachstums von Mikroorganismen.

## Revendications

1. Composé choisi parmi la 1-hydroxy-6-(3,5,5-triméthylhexyloxy)pyridin-2(1H)-one, la 1-hydroxy-6-octyloxypyridin-2(1H)-one et la 1-hydroxy-6-octyloxypyridine-2(1H)-thione.

2. Composé selon la revendication 1 choisi parmi la 1-hydroxy-6-(3,5,5-triméthylhexyloxy)pyridin-2(1H)-one et la 1-hydroxy-6-octyloxypyridin-2(1H)-one.

3. Procédé pour la fabrication de la 1-hydroxy-6-(3,5,5-triméthylhexyloxy)pyridin-2(1H)-one, **caractérisé par** les étapes de :
(a) la réaction du N-oxyde de 2,6-dichloropyridine et du 3,5,5-triméthyl-1-hexanol et d'une base, éventuellement en présence d'eau ou d'un solvant organique, à une température élevée, pour produire le N-oxyde de 2-chloro-6-(3,5,5-triméthylhexyloxy)pyridine et
(b) la réaction dudit N-oxyde de 2-chloro-6-(3,5,5-triméthylhexyloxy)pyridine avec une base pour produire la 1-hydroxy-6-(3,5,5-triméthylhexyloxy)pyridin-2(1H)-one.

4. Procédé pour la fabrication de la 1-hydroxy-6-octyloxypyridin-2(1H)-one, **caractérisé par** les étapes de :
(a) la réaction du N-oxyde de 2,6-dichloropyridine et du 1-octanol et d'une base, éventuellement en présence d'eau ou d'un solvant organique, à une température élevée, pour produire le N-oxyde de 2-chloro-6-octyloxypyridine et
(b) la réaction dudit N-oxyde de 2-chloro-6-octyloxypyridine avec une base pour produire la l-hydroxy-6-octyloxypyridin-2(1H)-one.

5. Méthode de préparation du composé 1-hydroxy-6-octyloxypyridine-2(1H)-thione, **caractérisée par** les étapes de :
(a) la réaction du N-oxyde de 2,6-dichloropyridine et du 1-octanol et d'une base, éventuellement en présence d'eau ou d'un solvant organique, à une température élevée, pour produire le N-oxyde de 2-chloro-6-octyloxypyridine et
(b) la réaction dudit N-oxyde de 2-chloro-6-octyloxypyridine avec un sel de sulfure ou d'hydrosulfure pour produire le composé 1-hydroxy-6-octyloxypyridine-2( 1H)-thione.

6. Composition antimicrobienne comprenant un composant fonctionnel choisi dans le groupe constitué par les peintures, les shampooings, les médicaments pour les soins de la peau, les fluides pour le travail des métaux, les compositions polymères, plus une quantité efficace en tant qu'antimicrobien d'un composé choisi parmi la 1-hydroxy-6-(3,5,5-triméthylhexyloxy)pyridin-2(1H)-one, la 1-hydroxy-6-octyloxypyridin-2(1H)-one et la 1-hydroxy-6-octyloxypyridine-2(1H)-thione.

7. Composé choisi parmi la 1-hydroxy-6-(3,5,5-triméthylhexyloxy)pyridin-2(1H)-one, la 1-hydroxy-6-octyloxypyridin-2(1H)-one et la 1-hydroxy-6-octyloxypyridine-2(1H)-thione pour son utilisation dans l'inhibition de la croissance des micro-organismes par la mise en contact desdits micro-organismes avec une composition **caractérisée en ce qu'**elle contient une quantité efficace en tant qu'antimicrobien dudit composé et au moins un composant choisi dans le groupe constitué par les savons, les adhésifs, les revêtements, les élastomères, les matériaux d'étanchéité, les shampooings, les médicaments pour les soins de la peau, les cosmétiques et les peintures.

8. Composition **caractérisée en ce qu'**elle contient une quantité efficace en tant qu'antimicrobien d'un composé choisi parmi la 1-hydroxy-6-(3,5,5-triméthylhexyloxy)pyridin-2(1H)-one, la 1-hydroxy-6-octyloxypyridin-2(1H)-one et la 1-hydroxy-6-octyloxypyridine-2(1H)-thione et au moins un composant choisi dans le groupe constitué par les savons, les adhésifs, les revêtements, les élastomères, les matériaux d'étanchéité, les shampooings, les médicaments pour les soins de la peau, les cosmétiques et les peintures, pour l'inhibition de la croissance de micro-organismes.
